# DEMANDE DE BREVET EUROPEEN

(11) **EP 4 635 470 A2**
(43) Date de publication de la demande: **22.10.2025**
(21) Numéro de dépôt: 25171454.9
(22) Date de dépôt: 18.04.2025
(51) Int. Cl.: A61K 6/891, A61K 6/77

(54) **MATERIAU UTILISABLE COMME RESINE DENTAIRE**

(30) Priorité: 18.04.2024 FR 2404034
(71) Demandeur: Samatech, 74950 Scionzier (FR)
(72) Inventeur: VINCI, Salvatore, 74950 SCIONZIER (FR)
(74) Mandataire: Denjean, Eric

(57) **Abrégé**

Matériau utilisable comme résine dentaire, caractérisé en ce que le matériau comprend :
- 30 % à 50 % de polyamide,
- 40 % à 60 % de fibres de renfort,
- 10 % à 30 % d'agent radio-opaque,
en poids, par rapport au poids total du matériau.

## Description

### Domaine technique de l'invention

La présente invention concerne le domaine dentaire et plus particulièrement un matériau utilisable comme résine dentaire. Elle se rapporte également à un bloc ou un disque pour usinage en CFAO comprenant le matériau. L'invention a également pour objet un élément prothétique dentaire obtenu à partir du bloc ou du disque, en particulier un inlay-core, tenon, bridge et armature. Elle concerne enfin un corps de scannage, un pilier ou un pivot comprenant le matériau dentaire.

### Art antérieur

La conception et fabrication assistée par ordinateur (CFAO) est une technologie aujourd'hui répandue dans le domaine de l'odontologie. Cette technique, également nommée CAD/CAM, permet aux chirurgiens-dentistes de concevoir des prothèses dentaires adaptées à chaque patient, directement dans leur cabinet.

Une telle pratique améliore la conception des prothèses dentaires et accroît globalement la vitesse de traitement des patients.

Les prothèses dentaires sont usinées à partir d'un bloc ou d'un disque, qui sont les deux types de préformes les plus communes.

Les blocs ont généralement une forme sensiblement parallélépipédique. Ils sont destinés à être fixés à la machine d'usinage au moyen d'une tige de maintien solidaire, ou non, du bloc. Les documents EP3395289 et FR3058630 décrivent notamment des blocs pour usinage en CFAO d'élément prothétique dentaire, tel que notamment inlay-core.

Les disques ont une forme circulaire ou ovale.

Les matériaux employés pour la conception des blocs ou des disques doivent être biocompatibles, pour éviter tout rejet, et avoir une résistance mécanique suffisante pour supporter les contraintes exercées par/sur les dents.

Les matériaux céramiques ont souvent été choisis pour leur apparence similaire à celles des dents ainsi que pour leurs propriétés mécaniques.

Les métaux peuvent également être utilisés.

Les matériaux composites comprenant des polymères représentent un bon compromis entre rigidité et biocompatibilité : les résines (méth)acrylique, les polyesters, les vinylesters ou les époxy sont fréquemment employés dans la fabrication de tels matériaux. En particulier, le polyétheréthercétone (PEEK) est un matériau très utilisé pour la fabrication de prothèses. Ce thermoplastique semi-cristallin est rigide et résiste efficacement à l'usure et à l'abrasion. Cependant, son prix est généralement élevé, il est donc recherché des alternatives au PEEK.

Les prothèses dentaires étant soumises à des contrôles réguliers par le praticien, notamment au moyen de radiographies, les matériaux sélectionnés doivent être radio-opaques, autrement dit ils doivent empêcher le passage des rayons X.

De tels matériaux sont plus facilement visibles sur une radiographie. Ils y apparaissent comme blancs ou gris clair.

Il est donc essentiel de mettre en œuvre des matériaux utilisables comme résine dentaire présentant un bon compromis entre la résistance mécanique et la radio-opacité.

Par ailleurs, il est connu d'utiliser des nappes de fibres de verre pour renforcer la résistance des disques CFAO. Ces nappes comprennent des réseaux de fibres de verre horizontales, parallèles les unes aux autres, comme le décrivent les documents EP2692312 et WO2012095878. Cependant, une telle structure de nappes de fibres longues n'est pas adaptée à l'usinage en bouche.

Le problème que se propose de résoudre l'invention est celui de s'affranchir des contraintes de l'état de la technique en proposant un matériau mécaniquement résistant et radio-opaque utilisable comme résine dentaire, qui puisse servir à la fabrication à la fois de disques et de blocs.

L'objectif de l'invention est également celui de mettre au point une résine qui soit injectable pour former des disques d'une épaisseur compatible avec l'usinage d'armature notamment.

### Exposé de l'invention

L'invention concerne en premier lieu un matériau utilisable comme résine dentaire. Ce matériau se caractérise en ce qu'il comprend :
- 30 % à 50 % de polyamide,
- 40 % à 60 % de fibres de renfort,
- 10 % à 30 % d'agent radio-opaque,
en poids, par rapport au poids total du matériau.

En d'autres termes, le Demandeur a mis au point un matériau qui remplit l'ensemble des critères évoqués précédemment, à savoir possibilité d'injecter le matériau, résistance mécanique et radio-opacité.

Le Demandeur a constaté qu'en dehors des intervalles mentionnés, la combinaison du polyamide, des fibres de renfort et de l'agent radio-opaque ne garantit pas la résistance mécanique, la radio-opacité et l'injectabilité du matériau.

Le matériau présente en outre l'avantage d'être usinable en bouche. En effet, contrairement aux matériaux de l'art antérieur, le matériau selon l'invention ne présente pas de fibres à sa périphérie une fois préusiné.

Les polyamides sont des polymères pouvant être obtenus par polycondensation entre un acide carboxylique et une amine. Ils ont généralement une structure semi-cristalline et sont particulièrement adaptés aux procédés d'injection pour leur mise en forme.

Le polyamide est, de manière préférentielle, le polyamide 6 et/ou le polyamide 6-6. Avantageusement, le polyamide est le polyamide 6-6. Le polyamide peut être le polyamide 10, le polyamide 11 ou le polyamide 12.

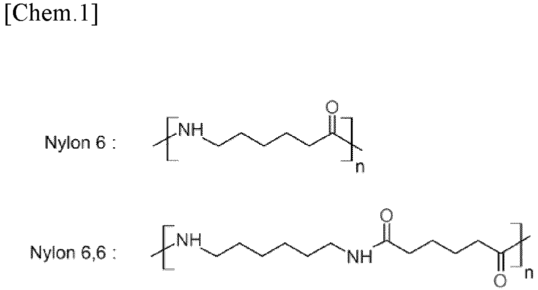

Les fibres de renfort ont, préférentiellement, une longueur inférieure à 5 mm, de préférence comprise entre 4 et 5 mm. Les fibres de cette longueur sont particulièrement résistantes aux contraintes mécaniques comme la compression. Du fait de leur taille, ces fibres sont dispersées dans la matière et renforcent ainsi sa résistance mécanique, rendant inutile la présence de nappes horizontales superposées dont les inconvénients ont été exposés précédemment.

Les fibres de renfort sont avantageusement choisies dans le groupe comprenant les fibres de verre, de kevlar, d'aramide, de carbone, avantageusement les fibres de verre.

Les fibres de verre sont particulièrement résistantes aux chocs et sont chimiquement inertes. Elles peuvent comprendre du SiO₂, du Al₂O₃, et/ou du CaO.

Les fibres de kevlar possèdent une structure cristalline et ont de bonnes propriétés mécaniques à la traction mais sont moins résistantes vis-à-vis de la compression que les fibres de verre.

Les fibres d'aramide sont résistantes mais leur usinage peut être difficile.

Les fibres de carbone présentent la meilleure résistance à la compression mais elles sont plus coûteuses et ont une couleur noire non compatible avec une application dentaire.

De manière avantageuse, l'agent radio-opaque comprend du baryum et/ou de l'iode, de préférence du baryum.

L'agent radio-opaque est préférablement le sulfate de baryum.

En pratique, le matériau comprend au moins 20 %, 25 %, 30 %, 35 %, 40 % voire même 45 % de polyamide en poids, par rapport au poids total du matériau.

Le matériau peut comprendre moins de 65 %, 60 %, 55 %, 50 % voire même 49 % de polyamide en poids, par rapport au poids total du matériau.

En pratique, le matériau comprend au moins 30 %, 35 %, 40 %, 45 %, voire même 50 % de fibres de renfort en poids, par rapport au poids total du matériau.

Le matériau peut comprendre moins de 70 %, 65 %, 60 %, voire même 55 %, de fibres de renfort en poids, par rapport au poids total du matériau.

En pratique, le matériau comprend au moins 5 %, 7 %, 10 %, 15 %, voire même 20 % d'agent radio-opaque en poids, par rapport au poids total du matériau.

Le matériau peut comprendre moins de 40 %, 35 %, 30 %, voire même 25 % d'agent radio-opaque en poids, par rapport au poids total du matériau.

Dans un mode de réalisation préféré, le matériau comprend :
- 38 % à 42 % de polyamide 6-6,
- 48 % à 52 % de fibres de verre de longueur inférieure à 5 mm,
- 10 % à 14 % de sulfate de baryum,
en poids, par rapport au poids total du matériau.

Le matériau peut avoir une apparence translucide ou être teintée par le praticien dentaire pour s'accorder avec la coloration de la dentition du patient.

La présente invention concerne également un bloc ou un disque pour usinage en CFAO d'un élément prothétique dentaire comprenant le matériau selon l'invention.

En particulier, le bloc ou le disque pour usinage en CFAO d'un élément prothétique dentaire est constitué du seul matériau selon l'invention. Le matériau comprend du polyamide, des fibres de renfort, et un agent radio-opaque.

Le bloc comprend une partie usinable, de forme sensiblement parallélépipédique, ainsi qu'une tige de maintien destinée à fixer le bloc sur une machine d'usinage en CFAO.

Avantageusement, la partie usinable et la tige de maintien sont réalisées en une seule pièce ou en deux pièces distinctes pouvant être fixées l'une à l'autre.

Selon l'invention, au moins la partie usinable comprend le matériau selon l'invention.

Le matériau selon l'invention est injectable dans des conditions de température et de refroidissement qui permettent d'obtenir un bloc ou un disque de dimension compatible avec l'usinage en CFAO.

En particulier, la matière doit être injectable à une épaisseur de 14 à 18 mm pour l'obtention d'un disque.

Le bloc ou le disque est fabriqué par moulage par injection, selon un mode de réalisation particulier.

L'invention vise également un élément prothétique dentaire obtenu par usinage en CFAO à partir d'un bloc ou un disque comprenant le matériau selon l'invention.

Avantageusement ce bloc ou ce disque est constitué du matériau selon l'invention.

L'élément prothétique dentaire peut être un inlay-core, un tenon, une armature, un bridge ou une couronne.

Un inlay-core est généralement constitué d'une tige aussi appelée tenon, surmontée d'un moignon de dent reconstituée. Le rôle de l'inlay-core est de servir de point d'ancrage à une prothèse dentaire. Les éléments prothétiques dentaires tels que les couronnes ou les bridges peuvent venir s'insérer au niveau du moignon de l'inlay-core.

Une couronne est une dent artificielle qui recouvre une dent abîmée.

Le bridge permet de remplacer une ou plusieurs dents manquantes, en s'appuyant sur les dents adjacentes. Il peut être composé de plusieurs couronnes solidarisées.

L'armature est une structure permettant la fixation des couronnes ou des bridges.

Le tenon correspond à la tige de maintien de l'inlay-core.

Selon un mode de réalisation, un mordançage peut être effectué sur le moignon avant la mise en place du tenon. Le mordançage est une technique qui permet d'améliorer l'adhérence des prothèses dentaires à la structure dentaire. Elle consiste en l'application d'un acide, généralement l'acide phosphorique ou l'acide fluorhydrique, sur le moignon. L'attaque de l'acide sur l'émail et la dentine aide à faire pénétrer la résine dans la matrice collagène de la dent. L'acide est rincé et un composé adhésif peut être appliqué au niveau du moignon. Le composé adhésif peut être photopolymérisé par exposition au rayonnement ultraviolet. Le tenon est alors mis en place dans le moignon.

L'invention a également pour objet un corps de scannage, un pilier ou un pivot comprenant le matériau selon l'invention.

Un pilier est un dispositif implantaire qui se visse généralement sur un implant substituant la racine d'une dent extraite, pour la fixation d'une couronne.

Un corps de scannage permet d'indiquer la position et l'orientation d'un implant dentaire à l'aide d'une caméra numérique. Il peut être fixé sur l'implant ou sur le pilier.

Un pivot comprend un inlay-core et une couronne dentaire. C'est une technique de restauration permettant de garder la racine d'une dent dévitalisée et ainsi d'éviter la pose d'un implant dentaire.

### Brève description des dessins

La manière dont l'invention peut être réalisée et les avantages qui en découlent ressortiront mieux de l'exemple de réalisation qui suit, donné à titre indicatif et non limitatif, à l'appui des figures annexées.
- La figure 1 est une radiographie d'un inlay-core obtenu à partir d'un bloc 1 dont la fabrication est détaillée ci-après.
- La figure 2 est une radiographie d'un inlay-core obtenu à partir d'un bloc 2 dont la fabrication est détaillée ci-après.
- La figure 3 est une radiographie d'un inlay-core obtenu à partir d'un bloc 3 dont la fabrication est détaillée ci-après.

### Exemples de réalisation de l'invention

### Exemple 1 : Fabrication de blocs pour usinage en CFAO

On fabrique trois blocs pour usinage en CFAO comprenant le matériau selon l'invention, selon le protocole suivant.

On injecte un matériau constitué de 39 % de polyamide 6-6, 48 % de fibres de verre de longueur inférieure à 5 mm et 13 % de sulfate de baryum en poids. On forme le bloc 1 par injection puis moulage dans les conditions suivantes.

Le moulage par injection est réalisé à l'aide d'une presse SELECT^{®} 200T. Le mélange est chauffé et fondu à 250°C, puis injecté à 90°C dans un moule ayant une température de 70°C. Le moule est refroidi et le bloc est récupéré.

Ce même procédé est mis en œuvre pour la fabrication des blocs 2 et 3.

Le bloc 2 est obtenu par injection et moulage d'un matériau constitué de 40 % de polyamide 6-6, 50 % de fibres de verre de longueur inférieure à 5 mm et 10% de sulfate de baryum, en poids.

Le bloc 3 est obtenu par injection et moulage d'un matériau constitué de 38 % de polyamide 6-6, 52 % de fibres de verre de longueur inférieure à 5 mm et 10 % de sulfate de baryum.

Les compositions des blocs sont détaillées dans le Tableau 1, en pourcentage massique par rapport au poids total du matériau.

**[Tab.1]**

| | Bloc 1 | Bloc 2 | Bloc 3 |
|---|---|---|---|
| Polyamide 6-6 (%) | 39 | 40 | 38 |
| Fibres de verre (%) | 48 | 50 | 52 |
| Sulfate de baryum (%) | 13 | 10 | 10 |

### Exemple 2 : Fabrication de disques pour usinage en CFAO

On fabrique trois disques pour usinage en CFAO comprenant le matériau selon l'invention, selon le protocole suivant.

Ainsi, le disque 1 est mis en forme par moulage à injection d'un matériau constitué de 39 % de polyamide 6-6, 48 % de fibres de verre de longueur inférieure à 5 mm et 13 % de sulfate de baryum en poids.

Le moulage par injection est réalisé à l'aide d'une presse SELECT 200T. Le mélange est chauffé et fondu à 250°C, puis injecté à 90°C dans un moule ayant une température de 70°C. Le moule est refroidi pendant quelques minutes et le disque est récupéré.

Ce même procédé est mis en œuvre pour la fabrication des disques 2 et 3.

Le disque 2 est obtenu à partir d'un matériau constitué de 40 % de polyamide 6-6, 50 % de fibres de verre de longueur inférieure à 5 mm et 10% de sulfate de baryum, en poids.

Le disque 3 est obtenu à partir d'un matériau constitué 38 % de polyamide 6-6, 52 % de fibres de verre de longueur inférieure à 5 mm et 10 % de sulfate de baryum.

Les trois disques ont un diamètre de 98 mm et une épaisseur de 14 mm.

Les compositions des disques sont détaillées dans le Tableau 2, en pourcentage massique par rapport au poids total du matériau.

**[Tab.2]**

| | Disque 1 | Disque 2 | Disque 3 |
|---|---|---|---|
| Polyamide 6-6 (%) | 39 | 40 | 38 |
| Fibres de verre (%) | 48 | 50 | 52 |
| Sulfate de baryum (%) | 13 | 10 | 10 |

### Exemple 3 : Radio-opacité

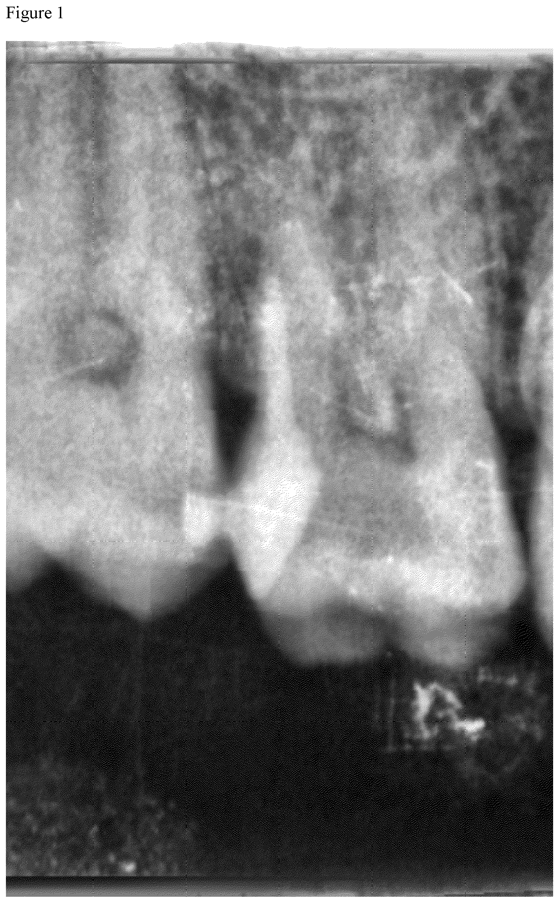

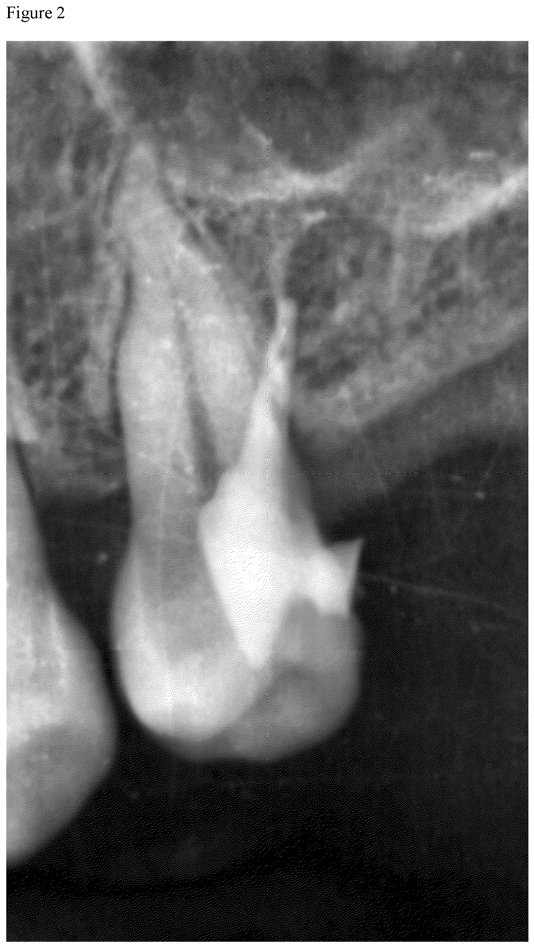

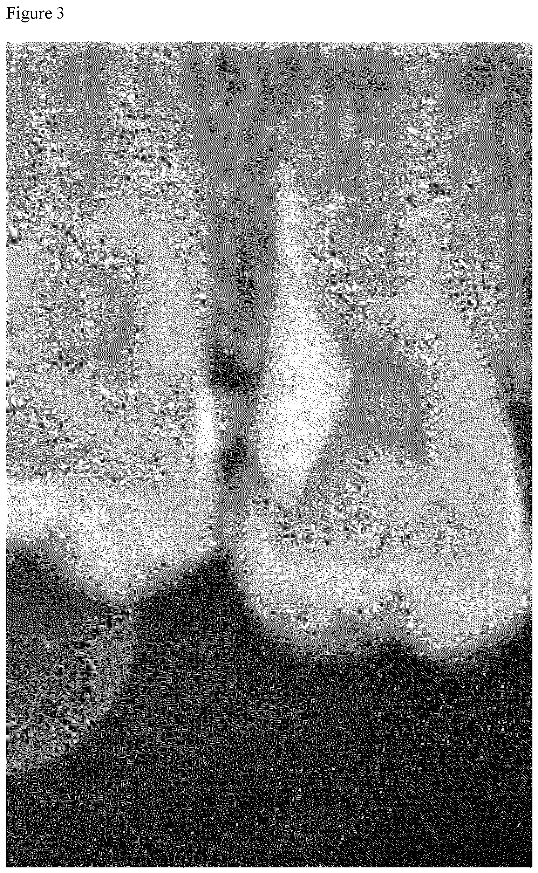

La figure 1, la figure 2 et la figure 3 montrent que pour chacun des matériaux utilisés, l'élément prothétique dentaire implanté présente une radio opacité satisfaisante.

## Revendications

1. Matériau utilisable comme résine dentaire, **caractérisé en ce que** le matériau comprend :
- 30 % à 50 % de polyamide,
- 40 % à 60 % de fibres de renfort ayant une longueur inférieure à 5 mm, de préférence comprise entre 4 et 5 mm,
- 10 % à 30 % d'agent radio-opaque,
en poids, par rapport au poids total du matériau.

2. Matériau selon la revendication 1, **caractérisé en ce que** le polyamide est le polyamide 6 et/ou le polyamide 6-6, avantageusement le polyamide 6-6.

3. Matériau selon l'une des revendications 1 ou 2, **caractérisé en ce que** les fibres de renfort sont choisies dans le groupe comprenant les fibres de verre, de kevlar, d'aramide, de carbone, avantageusement les fibres de verre.

4. Matériau selon l'une des revendications 1 à 3, **caractérisé en ce que** l'agent radio-opaque est le sulfate de baryum.

5. Matériau selon l'une des revendications 1 à 4, **caractérisé en ce qu'**il comprend :
- 38 % à 42 % de polyamide 6-6,
- 48 % à 52 % de fibres de verre de longueur inférieure à 5 mm,
- 10 % à 14 % de sulfate de baryum,
en poids, par rapport au poids total du matériau.

6. Bloc ou disque pour usinage en CFAO d'un élément prothétique dentaire, **caractérisé en ce qu'**il comprend le matériau selon l'une des revendications 1 à 5.

7. Bloc ou disque pour usinage en CFAO d'un élément prothétique dentaire, **caractérisé en ce qu'**il est constitué du matériau selon l'une des revendications 1 à 5.

8. Élément prothétique dentaire obtenu par usinage en CFAO d'un bloc ou disque selon l'une des revendications 6 ou 7, **caractérisé en ce que** ledit élément prothétique dentaire est un inlay-core, un tenon, une armature, un bridge ou une couronne.

9. Corps de scannage, pilier ou pivot comprenant le matériau selon l'une des revendications 1 à 5.
